# FASCICULE DE BREVET EUROPEEN

(11) **EP 2 813 261 B1**
(45) Date de publication et mention de la délivrance du brevet: **20.07.2016**
(21) Numéro de dépôt: 14166138.9
(22) Date de dépôt: 28.04.2014
(51) Int. Cl.: A61N 1/36

(54) **Dispositif médical implantable actif de thérapie neuronale avec stimulation stochastique du nerf vague**
Aktive implantierbare medizinische Vorrichtung zur neuronalen Behandlung mit stochastischer Stimulation des Vagusnervs
Active implantable medical device for nerve therapy with stochastic stimulation of the vagus nerve

(30) Priorité: 11.06.2013 FR 1355384
(43) Date de publication de la demande: 17.12.2014
(73) Titulaire: Sorin CRM SAS, 92140 Clamart Cedex (FR)
(72) Inventeur: Legay, Thierry, 91640 Fontenay Les Briis (FR); Blumstein, Hervé, 28130 Mévoisins (FR)
(74) Mandataire: Dupuis-Latour, Dominique

(56) Documents cités:
- EP-A1- 1 897 586
- US-A1- 2007 027 486
- US-A1- 2011 009 923
- US-A1- 2011 190 569
- US-A1- 2013 066 392
- US-B1- 7 076 308

## Description

L'invention concerne les "dispositifs médicaux implantables actifs" tels que définis par la directive 90/385/CEE du 20 juin 1990 du Conseil des communautés européennes, plus précisément les implants permettant de délivrer des thérapies de stimulation du nerf vague, dites thérapies VNS (*Vagus Nerve Stimulation*)*.*

La stimulation du système nerveux est une thérapie reconnue à l'égard de très nombreux troubles tels que l'épilepsie, la douleur, l'insuffisance cardiaque, l'apnée, l'obésité, etc.

Les dispositifs utilisés à cet effet comprennent une sonde pourvue d'une électrode implantée sur le nerf vague et un générateur délivrant des impulsions VNS sur cette électrode. Si le nerf vague doit en outre être stimulé de façon synchrone avec le rythme cardiaque, le dispositif peut comporter en outre une ou plusieurs sondes cardiaques permettant le recueil des ondes de dépolarisation du myocarde.

Dans tous les cas, la thérapie VNS consiste à ajouter un signal arbitraire (les impulsions VNS) se superposant aux signaux naturellement véhiculés par le système nerveux, organisé en boucle fermée. Cette stimulation du nerf vague pourra agir en efférence, directement vers un organe, ou en afférence, vers le cerveau pour influer sur le système nerveux central : le signal arbitraire constitué par les impulsions VNS sera alors interprété par le système nerveux central comme une sollicitation que ce dernier va tenter de compenser pour s'y opposer, et empêcher de produire ainsi les effets attendus.

Le profil de stimulation du nerf vague est composé de salves (ou trains) d'impulsions répétitives, produites pendant une période dite "d'activité", suivie d'une période "d'inactivité" durant laquelle la stimulation n'est plus délivrée. L'énergie de chaque salve est déterminée par la largeur, l'amplitude, le nombre, la fréquence et la morphologie des impulsions, paramètres qui sont tous ajustés par un paramétrage approprié du générateur d'impulsions VNS.

Le problème abordé par l'invention est lié au fait qu'une thérapie neuronale par stimulation VNS, si elle se révèle efficace au début de son application, semble toutefois souvent perdre cette efficacité au cours du temps (après quelques semaines) du fait de phénomènes de compensation provenant de la création d'une boucle de contrôle physiologique.

L'idée de base de l'invention consiste à appliquer une stimulation VNS à un patient en modulant chaque salve d'impulsions de manière à induire des variations de l'énergie délivrée par cette salve, ainsi que de l'intervalle entre impulsions.

Cette variabilité artificiellement induite de la stimulation VNS peut être de type stochastique, c'est-à-dire non déterministe, afin d'éviter la compensation de l'excitation du nerf vague par une boucle physiologique impliquant le système nerveux central, phénomène qui aurait pour effet de réduire les effets bénéfiques résultant de la thérapie VNS.

Ainsi, le US 2007/0027486 A1 décrit un générateur VNS dans lequel l'énergie délivrée est modulée d'une impulsion à la suivante, ou d'une salve à la suivante, en faisant varier de façon aléatoire la largeur et l'amplitude des impulsions, ainsi que l'intervalle de temps entre impulsions successives, ou encore l'intensité du courant délivré par l'impulsion. Mais un tel système requiert des systèmes complexes de contrôle des divers paramètres de fonctionnement du générateur (courant, séquencement, etc.) et rend difficile à contrôler l'énergie moyenne délivrée au patient, audelà de la modulation aléatoire.

Selon l'invention, la modulation stochastique est obtenue en contrôlant à chaque émission d'une salve d'impulsions la délivrance de chacune des impulsions de cette salve par une fonction aléatoire ou pseudo-aléatoire de type "pile ou face" appliquée à la délivrance de chacune des impulsions de chacune des salves, ce qui évite toute compensation potentielle par un mécanisme physiologique.

L'énergie VNS délivrée sur le nerf à chaque cycle cardiaque variera ainsi, de façon aléatoire, théoriquement entre zéro (aucune impulsion délivrée) et un maximum (la totalité des impulsions initialement prévues de la salve étant délivrées).

De ce fait, à chaque salve d'impulsions est appliquée sur le nerf vague une énergie différente, non prévisible, qui provoque par voie de conséquence une modulation imprévue de la thérapie VNS, évitant à une boucle physiologique de s'y opposer systématiquement. L'intervalle entre deux impulsions successives effectivement délivrées varie également de manière imprévisible.

De cette façon, on peut obtenir un effet durable de la thérapie neuronale car la compensation par une boucle de contrôle physiologique n'est plus possible, ou rendue beaucoup plus difficile.

Plus précisément, l'invention propose un dispositif médical implantable actif de thérapie neuronale par stimulation du nerf vague tel que divulgué notamment par le US 2007/0027486 A1 précité, c'est-à-dire comprenant un générateur, apte à produire des salves de N impulsions de stimulation VNS générées en succession pendant des périodes respectives d'activité, les périodes d'activité étant séparées par des périodes intercalaires d'inactivité durant lesquelles aucune stimulation n'est délivrée, et des moyens de modulation stochastique des salves, aptes à contrôler séparément la délivrance de chacune des N impulsions VNS de chaque salve, de manière à faire varier aléatoirement l'énergie de stimulation VNS contenue dans cette salve et l'intervalle entre impulsions successives et contrer ainsi l'apparition d'une boucle de compensation physiologique.

De façon caractéristique de l'invention, les moyens de modulation stochastique comprennent un générateur pseudo-aléatoire binaire de type "pile ou face", délivrant en sortie un résultat binaire aléatoire '0' ou '1' pour chaque impulsion VNS susceptible d'être appliquée, et des moyens inhibiteurs, aptes à commander respectivement l'inhibition ou l'autorisation de la délivrance de l'impulsion VNS en fonction dudit résultat binaire aléatoire, pour chacune des impulsions de chacune des salves successives, de manière à faire varier aléatoirement le nombre d'impulsions VNS délivrées dans une salve.

De préférence, les amplitudes respectives des impulsions VNS délivrées par le générateur sont toutes égales, de même que leurs largeurs respectives, ainsi que les intervalles de temps respectifs séparant les instants d'application de deux impulsions VNS successives susceptibles d'être délivrées par le générateur à chaque salve, que leur délivrance par le générateur soit on non inhibée par les moyens de modulation stochastique.

La fréquence de récurrence des impulsions VNS susceptibles d'être délivrées par le générateur peut notamment être comprise entre 15 et 50 Hz.

La durée des périodes d'activité est de préférence constante, et comprise entre 15 et 60 secondes. De même, la durée des périodes d'inactivité est de préférence constante, et comprise entre 2 et 10 minutes.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une vue générale de présentation du dispositif de l'invention, montrant le générateur et le nerf vague ainsi que la sonde utilisée.
La Figure 2 est une vue schématique par blocs correspondant aux principales fonctionnalités du générateur du dispositif de l'invention.
Les Figures 3a à 3c explicitent la manière dont est mise en oeuvre l'invention, au moyen de différents chronogrammes montrant sur une même ligne temporelle une succession de deux salves d'impulsions de stimulation VNS.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur VNS connu.

Un tel stimulateur comprend un microprocesseur programmable pourvu de circuits pour mettre en forme et délivrer des impulsions de stimulation à des électrodes implantées. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

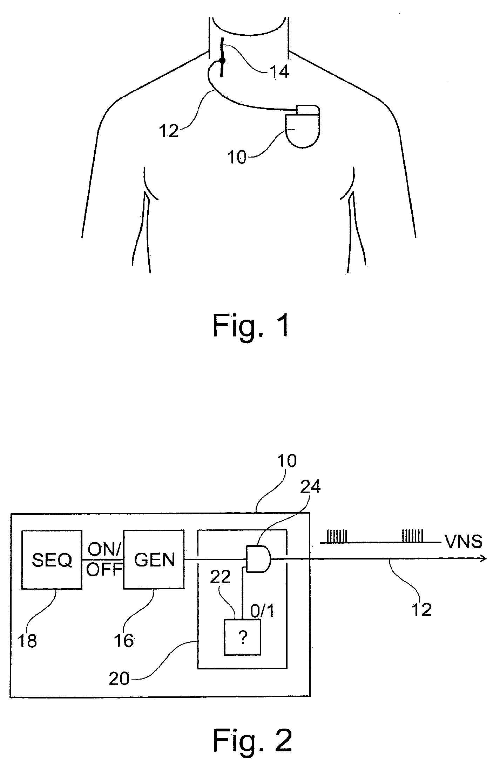

Sur la Figure 1, la référence 10 désigne le boitier d'un générateur implantable de stimulation du nerf vague. Cette stimulation est délivrée par une sonde 12 portant à sa partie distale une électrode implantée sur le nerf vague 14 et susceptible de stimuler celui-ci par application de salves d'impulsions produites par le générateur 10.

La Figure 2 illustre de façon schématique les principales fonctionnalités du générateur 10 du dispositif de l'invention.

Celui-ci comprend un circuit générateur 16 apte à produire des salves d'impulsions VNS délivrées au nerf vague via la sonde 12. Le circuit générateur 22 est commandé par un circuit séquenceur 18, qui pilote le générateur 16 de manière à délivrer des salves d'impulsions VNS pendant des périodes dites "d'activité" T_{ON}, entrecoupées de périodes "d'inactivité" T_{OFF}.

La fréquence de récurrence des impulsions produites par le générateur 16 est typiquement comprise entre 15 et 50 Hz, et les périodes d'activité et d'inactivité définies par le circuit séquenceur 18 sont typiquement comprises entre 15 à 60 secondes pour T_{ON} et 2 à 10 minutes pour T_{OFF}.

Un exemple représentatif, bien entendu non limitatif, de l'invention consiste à générer des impulsions à une fréquence de récurrence de 30 Hz pendant une durée T_{ON} = 30 secondes suivie d'une période d'inactivité T_{OFF} = 5 minutes.

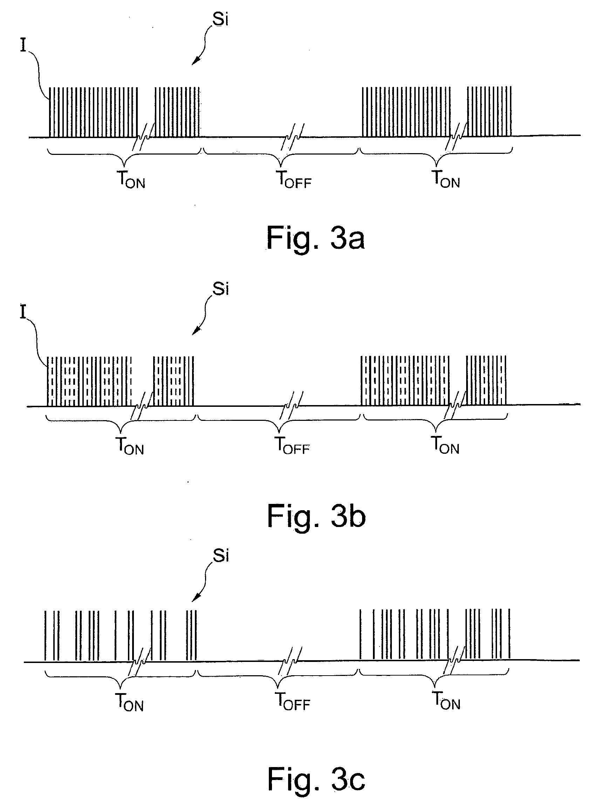

La Figure 3a illustre de telles salves Si d'impulsions VNS, constituées chacune d'une pluralité d'impulsions individuelles I, au nombre de 900 (30 Hz x 30 secondes) dans l'exemple cité plus haut. Les impulsions ont même amplitude et même largeur, de sorte que les impulsions délivrent toutes individuellement la même énergie de stimulation VNS. Par ailleurs, également dans cet exemple, l'intervalle entre deux impulsions I successives d'une même salve est un intervalle constant.

L'invention propose de moduler l'énergie des salves successivement appliquées au nerf vague en décidant, à chaque salve, de délivrer un nombre variable des impulsions initialement envisagées.

Cette technique est mise en oeuvre par le bloc 20 de la Figure 2, qui représente de manière schématique et symbolique les fonctions mises en oeuvre par l'invention, de préférence implémentées concrètement sous forme d'une routine du logiciel de pilotage du dispositif.

Le circuit de modulation 20 comporte un générateur pseudo-aléatoire binaire 22 de type "pile ou face", délivrant donc en sortie, pour chaque impulsion VNS susceptible d'être appliquée, une valeur '0' ou '1' qui commandera respectivement l'inhibition ou l'autorisation de la délivrance de l'impulsion VNS, et ce pour chacune des impulsions I de chacune des salves successives Si (fonction schématisée par la porte ET 24).

La fonction "pile ou face" peut être obtenue par exemple avec un algorithme de tirage pseudo-aléatoire d'un nombre sur N octets, le "pile" étant représenté par la valeur '0' d'un bit prédéterminé de ce nombre, et le "face" par la valeur '1' du bit de ce même nombre.

On peut utiliser par exemple un algorithme itératif définissant une suite S telle que Sₙ₊₁ =(Sₙ*16807) modulo 4294967296, avec S₀ choisi arbitrairement, par exemple S₀ étant une valeur représentant l'horloge interne d'un système ou une combinaison de cette horloge interne et d'un autre paramètre dépendant du temps. Le résultat '0' ou '1' du tirage de rang n+1 sera alors la valeur de l'un quelconque prédéterminé des bits de Sₙ₊₁.

Le résultat de cette fonction de modulation stochastique avec inhibition/autorisation de la délivrance de chacune des impulsions de chaque salve est illustré Figure 3b, avec les salves dont la délivrance a été inhibée par rapport à la Figure 3a représentées en tiretés.

L'énergie délivrée sur chaque salve Sᵢ va ainsi varier, de façon totalement imprévisible, entre un minimum et un maximum théoriques :
- le minimum correspond à une énergie nulle, situation où toutes les impulsions de la salve auraient été inhibées ;
- le maximum correspond au cas où aucune des impulsions n'aurait été inhibée.

On observe ainsi, comme on peut le voir Figure 3c, une variabilité très importante, induite d'une salve à la suivante, de l'intervalle entre impulsions successives effectivement délivrées, ainsi que de l'énergie de stimulation globalement délivrée par la salve, rendant impossible, ou tout au moins très difficile, la création d'une boucle physiologique de contrôle qui aurait pour effet de s'opposer à la thérapie neuronale que l'on souhaite appliquer au patient.

## Revendications

1. Un dispositif médical implantable actif de thérapie neuronale par stimulation du nerf vague, VNS, comprenant :
- un générateur (16), apte à produire des salves (Sᵢ) de N impulsions (I) de stimulation VNS générées en succession pendant des périodes respectives d'activité (T_{ON}), les périodes d'activité étant séparées par des périodes intercalaires d'inactivité (T_{OFF}) durant lesquelles aucune stimulation n'est délivrée ; et
- des moyens de modulation stochastique des salves (20), aptes à contrôler séparément la délivrance de chacune des N impulsions VNS de chaque salve, de manière à faire varier aléatoirement l'énergie de stimulation VNS contenue dans cette salve et l'intervalle entre impulsions successives et contrer ainsi l'apparition d'une boucle de compensation physiologique,
dispositif **caractérisé en ce que** les moyens de modulation stochastique comprennent :
- un générateur pseudo-aléatoire binaire (22) de type "pile ou face", délivrant en sortie un résultat binaire aléatoire '0' ou '1' pour chaque impulsion VNS susceptible d'être appliquée ; et
- des moyens inhibiteurs (24), aptes à commander respectivement l'inhibition ou l'autorisation de la délivrance de l'impulsion VNS en fonction dudit résultat binaire aléatoire, pour chacune des impulsions (I) de chacune des salves successives (Sᵢ), de manière à faire varier aléatoirement le nombre d'impulsions VNS délivrées dans une salve.

2. Le dispositif de la revendication 1, dans lequel les amplitudes respectives des impulsions VNS (I) délivrées par le générateur sont toutes égales.

3. Le dispositif de la revendication 1, dans lequel les largeurs respectives des impulsions VNS (I) délivrées par le générateur sont toutes égales.

4. Le dispositif de la revendication 1, dans lequel la fréquence de récurrence des impulsions VNS (I) successives susceptibles d'être délivrées par le générateur à chaque salve est constante sur la période d'activité, que la délivrance de ces impulsions par le générateur soit on non inhibée par les moyens de modulation stochastique.

5. Le dispositif de la revendication 4, dans lequel ladite fréquence de récurrence des impulsions VNS susceptibles d'être délivrées par le générateur est comprise entre 15 et 50 Hz.

6. Le dispositif de la revendication 1, dans lequel la durée desdites périodes d'activité (T_{ON}) est constante.

7. Le dispositif de la revendication 6, dans lequel la durée desdites périodes d'activité (T_{ON}) est comprise entre 15 et 60 secondes.

8. Le dispositif de la revendication 1, dans lequel la durée desdites périodes d'inactivité (T_{OFF}) est constante.

9. Le dispositif de la revendication 8, dans lequel la durée desdites périodes d'inactivité (T_{OFF}) est comprise entre 2 et 10 minutes.

## Patentansprüche

1. Aktive implantierbare medizinische Vorrichtung zur neuronalen Behandlung durch Vagusnervstimulation, VNS, umfassend:
- einen Generator (16), der geeignet ist, Bursts (Sᵢ) von N Impulsen (I) zur VNS-Stimulation zu produzieren, die nacheinander während jeweiliger Perioden der Aktivität (T_{ON}) generiert werden, wobei die Perioden der Aktivität durch Zwischenperioden der Inaktivität (stoff) getrennt sind, in denen keine Stimulation bereitgestellt wird; und
- Mittel zur stochastischen Modulation der Bursts (20), die geeignet sind, die Abgabe von jedem der N VNS-Impulse jedes Bursts derart separat zu steuern, dass die Energie der VNS-Stimulation, die in diesem Burst enthalten ist, und das Intervall zwischen aufeinanderfolgenden Impulsen zufällig variiert wird und somit dem Auftreten einer physiologischen Kompensationsschleife entgegengewirkt wird,
wobie die Vorrichtung, **dadurch gekennzeichnet ist, dass** die Mittel zur stochastischen Modulation Folgendes umfassen:
- einen binären Pseudozufallsgenerator (22) vom Typ "Kopf oder Zahl", der am Ausgang ein zufälliges Binärergebnis '0' oder '1' für jeden VNS-Impuls bereitstellt, der angewendet werden kann; und
- Hemmungsmittel (24), die geeignet sind, jeweils die Hemmung oder die Autorisierung der Abgabe des VNS-Impulses in Abhängigkeit von dem zufälligen Binärergebnis für jeden der Impulse (I) von jedem der aufeinanderfolgenden Bursts (Sᵢ) derart zu steuern, dass die Anzahl der VNS-Impulse, die in einem Burst abgegeben werden, zufällig variiert wird.

2. Vorrichtung nach Anspruch 1, wobei die jeweiligen Amplituden der VNS-Impulse (I), die von dem Generator abgegeben werden, alle gleich sind.

3. Vorrichtung nach Anspruch 1, wobei die jeweiligen Breiten der VNS-Impulse (I), die von dem Generator abgegeben werden, alle gleich sind.

4. Vorrichtung nach Anspruch 1, wobei die Wiederholungsfrequenz der aufeinanderfolgenden VNS-Impulse (I), die von dem Generator bei jedem Burst abgegeben werden können, über die Periode der Aktivität konstant ist, ob die Abgabe dieser Impulse durch den Generator durch die Mittel zur stochastischen Modulation gehemmt ist oder nicht.

5. Vorrichtung nach Anspruch 4, wobei die Wiederholungsfrequenz der VNS-Impulse, die von dem Generator abgegeben werden können, zwischen 15 und 50 Hz liegt.

6. Vorrichtung nach Anspruch 1, wobei die Dauer der Perioden der Aktivität (T_{ON}) konstant ist.

7. Vorrichtung nach Anspruch 6, wobei die Dauer der Perioden der Aktivität (T_{ON}) zwischen 15 und 60 Sekunden beträgt.

8. Vorrichtung nach Anspruch 1, wobei die Dauer der Perioden der Inaktivität (T_{OFF}) konstant ist.

9. Vorrichtung nach Anspruch 8, wobei die Dauer der Perioden der Inaktivität (T_{OFF}) zwischen 2 und 10 Minuten beträgt.

## Claims

1. An active implantable medical device for neural therapy by stimulation of the vagus nerve, VNS, comprising:
- a generator (16), adapted to produce bursts (Si) of N pulses (I) of VNS stimulation generated in succession during respective periods of activity (T_{ON}), the periods of activities being separated by intermediate periods of inactivity (T_{OFF}) during which no stimulation is delivered; and
- burst stochastic modulation means (20), adapted to control separately the delivery of each of the N VNS pulses of each burst, so as to randomly vary the energy of VNS stimulation contained in this burst and the interval between successive pulses and hence counter the appearance of a physiological compensation loop,
the device being **characterized in that** the stochastic modulation means comprise:
- a binary pseudo-random generator (22) of the "heads or tails" type, outputting a random binary result '0' or '1' for each VNS pulse liable to be applied; and
- inhibitor means (24), adapted to control the inhibition or authorization, respectively, of the delivery of the VNS pulse as a function of said random binary result, for each of the pulses (I) of each of the successive bursts (Si), so as to randomly vary the number of VNS pulses delivered in a burst.

2. The device of claim 1, wherein the respective amplitudes of the VNS pulses (I) delivered by the generator are all equal to each other.

3. The device of claim 1, wherein the respective widths of the VNS pulses (I) delivered by the generator are all equal to each other.

4. The device of claim 1, wherein the recurrence frequency of the successive VNS pulses (I) liable to be delivered by the generator at each burst is constant over the period of activity, whether the delivery of these pulses by the generator is inhibited or not by the stochastic modulation means.

5. The device of claim 4, wherein said recurrence frequency of the successive VNS pulses liable to be delivered by the generator is comprised between 15 and 50 Hz.

6. The device of claim 1, wherein the duration of said periods of activity (T_{ON}) is constant.

7. The device of claim 6, wherein the duration of said periods of activity (T_{ON}) is comprised between 15 and 60 seconds.

8. The device of claim 1, wherein the duration of said periods of inactivity (T_{OFF}) is constant.

9. The device of claim 8, wherein the duration of said periods of inactivity (To_{FF}) is comprised between 2 and 10 minutes.
